(19) Europäisches Patentamt
European Patent Office
Office européen des brevets

(11) Numéro de publication: **0 135 415**
**B1**

# (12) FASCICULE DE BREVET EUROPÉEN

(45) Date de publication du fascicule du brevet: **29.04.87**

(21) Numéro de dépôt: **84401520.6**

(22) Date de dépôt: **19.07.84**

(51) Int. Cl.⁴: **C 07 C 69/747**, C 07 C 67/327, C 07 C 154/00, C 07 C 59/42, C 07 D 491/048

## (54) Procédé de préparation d'esters d'alcoyle de l'acide cis chrysanthémique et nouveaux composés obtenus.

(30) Priorité: **21.07.83 FR 8312073**

(43) Date de publication de la demande: **27.03.85 Bulletin 85/13**

(45) Mention de la délivrance du brevet: **29.04.87 Bulletin 87/18**

(84) Etats contractants désignés: **CH DE GB IT LI NL**

(56) Documents cité:
**DE-A-2 639 777**
**GB-A-2 085 428**

**JOURNAL OF THE CHEMICAL SOCIETY, Chemical Communications, no. 22, 22 novembre 1972 H. GERLACH et al.: "Synthesis of olefins from sterically hindered alcohols by pyrolysis of thiocarbonate O-esters", pages 1215-1216**
**TETRAHEDRON LETTERS, vol. 23, no. 13, 1982, Pergamon Press Ltd. GB MAREK MAJEWSKI et al.: "Synthesis of pyrethroids via epoxyamide cyclization", pages 1343-1344**

(73) Titulaire: **ROUSSEL- UCLAF, 35, boulevard des Invalides, F-75007 Paris (FR)**

(72) Inventeur: **Franck- Neumann, Michel, 4, rue Andrieux, F-67000 Strasbourg (FR)**
Inventeur: **Sedrati, Madjid, 5, rue des Vignes, F-67990 Osthoffen (FR)**
Inventeur: **Vigneron, Jean- Pierre, 9, rue des Closeaux, F-91790 Boissy- sous- Saint- Yon (FR)**
Inventeur: **Bloy, Vincente, 15, avenue de Villars, F-75007 Paris (FR)**

(74) Mandataire: **Tonnellier, Marie- José, Département des Brevets ROUSSEL UCLAF B.P. no 9, F-93230 Romainville (FR)**

EP 0 135 415 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a pour objet un procédé de préparation d'esters d'alcoyle de l'acide cis chrysanthémique, racémiques ou optiquement actifs, ainsi que de nouveaux composés obtenus.

L'invention a ainsi notamment pour objet un procédé de préparation d'esters d'alcoyle de l'acide cis chrysanthémique, racémiques ou optiquement actifs, répondant à la formule (I):

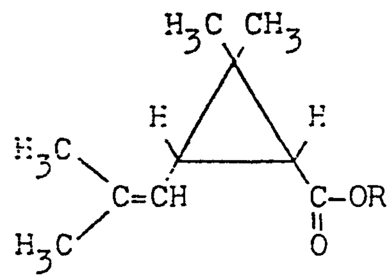

(I)

dans laquelle R représente un radical alcoyle renfermant de 1 à 6 atomes de carbone, caractérisé en ce que l'on traite un composé de formule (II):

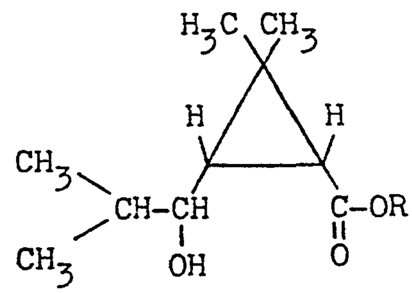

(II)

de configuration cis, racémique ou optiquement actif, dans laquelle R a la signification précitée, par un halogénothioformiate d'aryle de formule:

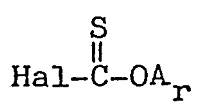

(III)

dans laquelle Hal représente un atome d'halogène et $A_r$ représente un radical aryle renfermant de 6 à 12 atomes de carbone, éventuellement substitué, pour obtenir un composé de formule (IV):

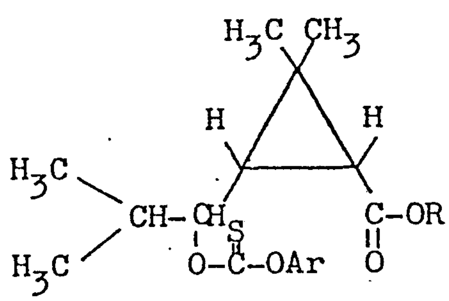

(IV)

de configuration cis, formule (IV) dans laquelle R et $A_r$ ont la signification précitée, que l'on chauffe, le cas échéant au sein d'un solvant organique, pour obtenir un composé de formule (I) attendu.

Dans la formule (I), R peut représenter un radical méthyle ou éthyle ou un radical propyle, butyle, pentyle ou hexyle, linéaire ou ramifié.

Dans la formule (III), Hal représente de préférence un atome de chlore et $A_r$ représente de préférence un radical phényle ou naphtyle. Lorsque le radical. $A_r$ est substitué, il s'agit de préférence d'un radical substitué par un ou plusieurs substituants alcoyles, renfermant de 1 à 3 atomes de carbone.

Le composé de formule (III) préféré est le chlorothioformiate de p-tolyle.

Dans des conditions préférentielles d'exécution du procédé de l'invention, on chauffe le composé de formule (IV) à une température de 100 à 150°C, au sein d'un solvant organique non polaire, lequel est, de préférence un dichloro ou un trichlorobenzène. Il est cependant également possible d'opérer en l'absence de

solvant.

Les composés de formule (II) sont connus et décrits, par exemple, dans Agr. Bio. Chem. 28(7) 456-66 (1964). Egalement selon l'invention, le composé de formule (II) de configuration cis, racémique ou optiquement actif peut encore être préparé selon un procédé caractérisé en ce que l'on traite le composé de formule (X):

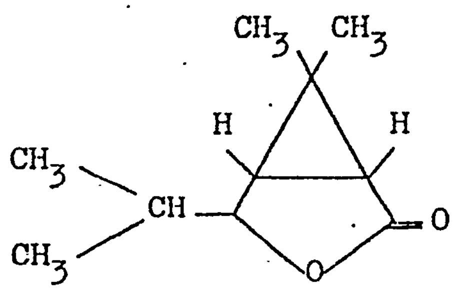

(X)

par un agent basique, puis par un agent d'estérification, pour obtenir le composé de formule (II).

Dans des conditions préféreritielles d'exécution du procédé de l'invention:

- le traitement par un agent basique du composé de formule (X) est effectué par un hydroxyde alcalin tel que la soude ou, de préférence, la potasse et l'on opère au sein d'un solvant hydro-alcoolique;

- l'estérification conduisant aux esters de formule (II) est effectuée in situ, sans isolement de l'acide issu de la saponification du composé de formule (X).

L'agent d'estérification conduisant aux composés de formule (II) peut être tout réactif connu de l'homme de métier pour conduire aux esters attendus. Lorsque l'on souhaite préparer l'ester méthylique, il es particulièrement commode d'utiliser le diazométhane. Lorsque l'on souhaite préparer d'autres esters d'alcoyle, les diazoalcanes correspondants peuvent être utilisés.

Le composé de formule (X) peut être préparé par un procédé selon lequel l'on fait réagir un composé de formule (V):

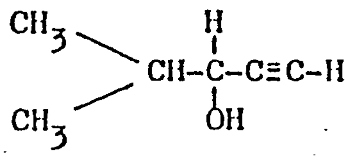

(V)

racémique ou optiquement actif, avec un réactif de carbonatation, pour obtenir un composé de formule (VI):

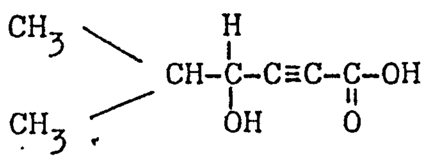

(VI)

que l'on soumet à une réduction sélective pour obtenir le composé de formule (VII):

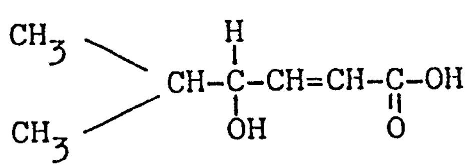

(VII)

que l'on cyclise pour obtenir la lactone de formule (VIII):

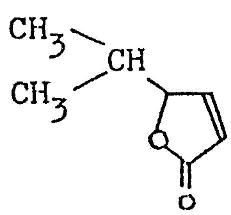

(VIII)

que l'on fait réagir avec le diazo-2-propane, pour obtenir un mélange de pyrazolines de formules ($IX_a$) et ($IX_b$):

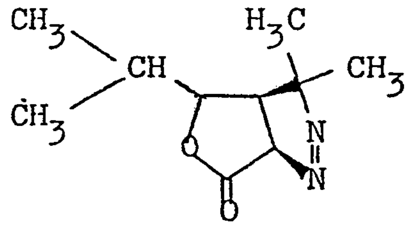 $(IX_a)$ 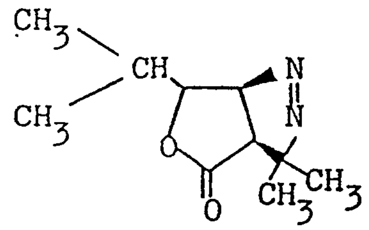 $(IX_b)$.

que l'on sépare, le cas échéant puis soumet le composé de formule $(IX_a)$ ou le composé de formule $(IX_b)$ ou leur mélange à une irradiation, en présence d'un sensibilisateur pour obtenir le composé de formule (X) attendu.

La carbonatation du composé de formule (V) est par exemple effectuée par action de gaz carbonique, en présence d'une base forte qui est de préférence, un alkyl lithien.

La réduction du composé de formule (VI) est par exemple effectuée par l'hydrogène en présence d'un catalyseur.

La cyclisation du composé de formule (VII) est par exemple effectuée soit spontanément, soit en présence d'un acide, lequel est, de préférence, un acide minéral.

L'irradiation des pyrazolines de formule $(IX_a)$ ou $(IX_b)$ ou de leur mélange, est par exemple effectuée à l'aide d'une lampe à vapeur de mercure, en présence d'un sensibilisateur tel que la benzophénone.

La synthèse des acides cis chrysanthémiques ou de leurs esters d'alcoyle présente à l'heure actuelle, un intérêt tout particulier, car ils sont utilisés notamment pour préparer des acides cyclopropane carboxyliques à chaîne dihalovinylique dont certains esters sont bien connus pour posséder une activité pesticide, notamment insecticide, remarquable.

Le procédé de la présente invention fournit une synthèse totale d'esters d'alcoyle d'acide cis chrysanthémique, racémiques ou optiquement actifs, ne comportant qu'un nombre restreint de stades, dont les rendements sont élevés. Cette synthése est stéréospécifique. En effet, la réaction d'addition et de cyclisation conduisant aux pyrazolines de formule $(IX_a)$ et $(IX_b)$ est stéréospécifique et les étapes suivantes ne provoquent aucune isomérisation.

Le composé de formule (II), puis les composés de formule (IV) et (I), de configuration cis, sont les seuls isomères obtenus.

En outre, le procédé de la présente invention présente un caractère tout à fait inattendu, compte-tenu de l'enseignement de l'art antérieur. En effet, plusieurs auteurs, parmi lesquels Matsui et Coll. Agr. Biol. Chem. 28 456 (1964), Ficini et Coll. Tet. Letters 1976, 2441 et Snieckus et Coll. Tet. Letters 1982, 1343 ne sont pas parvenus à déshydrater l'alcool de formule (II) de configuration cis. Le procédé de la présente invention permet cette déshydratation avec un rendement global qui peut dépasser 70 %.

Le composé de formule (V) racémique ou optiquement actif, est connu et décrit, par exemple, dans Tet. Letters n° 29 p. 2683 (1979).

Les exemples suivants illustrent l'invention sans toutefois la limiter.

**EXEMPLE 1**: (1S,cis) chrysanthémate de méthyle.

Stade A: Acide 4R-hydroxy 5-méthyl hex-2-ynoïque.

A 10,5 g de 3R-hydroxy 4-méthyl pent-2-yne dissous dans 180 $cm^3$ de tétrahydrofuranne refroidi à -78° C, on ajoute lentement 195,6 $cm^3$ d'une solution 1,3 M de butyllithium dans l'hexane. L'addition terminée, on ajoute, par petits morceaux, un excès de gaz carbonique solide. Il y a formation d'un précipité abondant. Après retour à la température ambiante, on ajoute 100 $cm^3$ d'eau pour dissoudre la masse colloïdale. La phase aqueuse est séparée de la phase organique, acidifiée avec de l'acide chlorhydrique concentré et extraite à l'éther. On évapore le solvant et obtient 21,05 g de produit attendu que l'on recristallise dans le benzène. On récupère ainsi 8,9 g de cristaux blancs. F = 80° C, $(\alpha)^{25}_D$ + 10,6° (c = 2 % dioxanne).

Stade B :5R-(1-méthyléthyl)-2 (5H) furanone.

7,62 g de l'acide obtenu au stade A, dissous dans 120 $cm^3$ de méthanol sont hydrogénés à la température et à la pression ordinaires en présence de 500 mg de palladium sur sulfate de baryum et de quelques gouttes de quinoléine. On arrête l'hydrogénation quand la quantité théorique d'hydrogène est fixée. On filtre le catalyseur et évapore le filtrat. Celui-ci est dissous dans 20 $cm^3$ d'éther; on ajoute à cette solution 2 $cm^3$ de HCL concentré et agite vigoureusement. La phase éthérée est séparée, lavée au bicarbonate de sodium et à l'eau. On sèche sur sulfate de sodium, évapore l'éther et distille le résidu. On obtient ainsi 5,70 g de produit attendu; Eb = 78-79° C/0,1 torr. $(\alpha)^{25}_D$ = -96° (c = 2 % dioxanne).

Stade C :4,4-diméthyl-6R-(1-méthyléthyl) 7-oxa 2,3-diazabicyclo-oct-2-èn-8-one (composé A) et 4,4-diméthyl-8R-(1-méthyléthyl) 7-oxa 2,3-diazabicyclo-oct-2-èn-6-one (composé B).

On ajoute par fractions de 5 ml, 25 ml d'une solution 3,3 M de diazo-2 propane dans le mélange éther-éthylbenzène préparé selon C. Dietrich-Buchecker et M. Franck-Neumann (Tetrahedron, 33, 745 (1977)) à une

solution de 5,30 g de produit obtenu au stade B, dans 20 ml d'éther, en maintenant la température à -10°C environ. Après 2 h. à température ambiante, on élimine l'éther à température ambiante. Le résidu est chromatographié sur silice en éluant avec de l'hexane à 20 % d'éther. On obtient ainsi une première fraction de 2,58 g de composé B cristallisé (F=78-82°C avec décomposition), suivie de 300 mg de mélange des composés A et B et une dernière fraction de 4,37 g de composé A sous forme de liquide incolore.

Stade D: (1S, 3R, 4R) 6,6-diméthyl 4-(1-méthyléthyl) 3-oxa bicyclo-hexan-2-one.

Une solution de 2,38 g de composé B obtenu ci-dessus et de 4,8 g de benzophénone dans 200 ml de benzène est irradiée avec une lampe à vapeur de mercure du type Philips HPK125 jusqu'à dégagement stoechiométrique total d'azote. Après évaporation du solvant, le résidu est chromatographié sur silice en éluant avec de l'hexane à 20 % d'éther. On recueille 1,28 g de lactone cyclopropanique attendue de pouvoir rotatoire $(\alpha)^{20}_D = +80°$ (chloroforme).

La photolyse dans les mêmes conditions de 4,30 g de composé A en présence de 9,0 g de benzophénone en solution dans 1 l de benzène conduit après dégagement total d'azote et isolement comme précédemment, à l'obtention de 3,30 g de la même lactone cyclopropanique que ci-dessus de pouvoir rotatoire $(\alpha)^{20}_D = +70°$ (chloroforme).

Stade E: (1S, 3R) 2,2-diméthyl3-(1-hydroxy 2-méthyl) propyl 1-cyclopropane carboxylate de méthyle.

Une solution de 4,2 g de lactone cyclopropanique obtenue au stade D dans 55 ml d'éthanol est mélangée avec une solution de 11,3 g de potasse dans 30 ml d'eau. Après 48 heures à température ambiante et élimination de la majeure partie de l'éthanol sous pression réduite, on dilue avec de l'eau et lave la solution aqueuse avec de l'éther. La phase aqueuse est ensuite acidifiée avec de l'acide chlorhydrique à 10 % et extraite à l'éther. On sèche l'extrait organique et traite immédiatement avec une solution éthérée de diazométhane en léger excès. Après une heure à température ambiante on évapore le solvant sous pression réduite. On obtient 5,0 g d'alcool-ester attendu utilisé tel quel dans la suite de la synthèse.

Stade F: (1S, 3R) 2,2-diméthyl 3-/1-(4-méthyl phénoxy carbonothioyloxy)-2-méthyl-propyl/ 1-cyclopropane carboxylate de méthyle.

On ajoute goutte à goutte 2,4 ml de chlorothioformiate de 0 para tolyle à un mélange de 2,80 g d'alcool-ester obtenu au stade E et de 1,5 ml de pyridine dans 40 ml de chlorure de méthylène. Après 48 heures à température ambiante, le mélange réactionnel est dilué avec 200 ml d'éther et lavé avec de l'eau. Après séchage et élimination des solvants, le résidu est chromatographié sur silice en éluant avec de l'hexane à 5% d'éther. On obtient ainsi 2,34 g de produit attendu.

Stade G: (1S cis) chrysanthémate de méthyle.

Une solution de 1,725 g de produit obtenu au stade F dans 20 ml de trichloro-1,2,4-benzène est chauffée lentement jusqu'à 140°C, puis maintenue pendant un quart d'heure à cette température. Après refroidissement, la solution est chromatographiée sur silice et éluée avec de l'hexane à 2 % d'éther. On recueille 692 mg de cis-chrysanthémate de méthyle attendu présentant un $(\alpha)^{20}_D$ de -60,5° (benzène) de configuration (1S, 3R).

**EXEMPLE 2**: (1R, cis) chrysanthémate de méthyle.

On opère de manière analogue à celle décrite à l'exemple 1 en utilisant au départ le 3S-hydroxy 4-méthyl pent-2-yne. On obtient après la même succession de stades, le (1R, cis) chrysanthémate de méthyle dont l'$\alpha^{20}_D$ est de +66,5° (benzène).

**EXEMPLE 3**: cis chrysanthémate de méthyle.

Stade A: 4,4-diméthyl-6(1-méthyléthyl)7-oxa 2,3-diazabicyclo-oct-2-èn-8-one (composé C) et 4,4-diméthyl-8(1-méthyléthyl)7-oxa 2,3-diazabicyclo-oct-2-èn-6-one (composé D).

On ajoute par fractions de 5 ml, 40 ml d'une solution 3 M de diazo-2 propane dans le mélange éther-éthylbenzène préparé selon C. Dietrich-Buchecker et M. Franck-Neumann (Tetrahedron, 33, 745 (1977)) à une solution de 7,70 g de 5(RS) (1-méthyléthyl)-2(5H)furanone obtenue comme décrit à l'exemple 1 en utilisant au départ un 3(RS) hydroxy 4-méthyl pent-2-yne, dans 20 ml d'éther en maintenant la température à -10°C environ. Après 2 h. à température ambiante, on élimine l'éther à température ambiante. Le résidu est chromatographié sur silice en éluant avec de l'hexane à 20 % d'éther. On obtient ainsi une fraction de 4,45 g de composé D cristallisé (F=68-69°C) et une fraction de 7,25 g de composé C sous forme de liquide incolore.

Stade B: 6,6-diméthyl 4-(1-méthyléthyl) 3-oxa bicyclo-hexan-2-one.

Une solution de 3,50 g de composé D obtenu ci-dessus et de 7,00 g de benzophénone dans 1 litre de benzène est irradiée avec une lampe à vapeur de mercure du type Philips HPK125 jusqu'à dégagement stoechiométrique total d'azote. Après évaporation du solvant, le résidu est chromatographié sur silice en éluant avec de l'hexane à 5 puis 10 % d'éther. On recueille 2,73 g de lactone cyclopropanique attendue (Eb=47-48°C/0,05 mmHg).

La photolyse dans les mêmes conditions de 4,30 g de composé C en présence de 9,0 g de benzophénone en solution dans 1 l de benzène conduit après dégagement total d'azote et isolement comme précédemment, à

l'obtention de 3,30 g de la même lactone cyclopropanique.

Stade C: 2,2-diméthyl 3(1-hydroxy 2-méthyl) propyl 1-cyclopropane carboxylate de méthyle cis.

Une solution de 2,7 g de lactone cyclopropanique obtenue ci-dessus dans 40 ml d'éthanol est mélangée avec une solution de 7,5 g de potasse dans 20 ml d'eau. Après 48 heures à température ambiante et élimination de la majeure partie de l'éthanol sous pression réduite, on dilue avec de l'eau et lave la solution aqueuse avec de l'éther. La phase aqueuse est ensuite acidifiée avec de l'acide chlorhydrique à 10 % et extraite à l'éther. On sèche l'extrait organique, concentre et traite immédiatement l'alcool-acide (2,9 g) par une solution éthérée de diazométhane en léger excès. Après une heure à température ambiante on évapore le solvant sous pression réduite. On obtient 3,10 g d'alcool-ester attendu (F = 58-59°C après recristallisation dans le mélange éther éthylique-hexane).

Stade D: 2,2-dimethyl 3-/1-(4-methyl phénoxy carbonothioyloxy) 2-méthyl-propyl/ 1-cyclopropane carboxylate de méthyle cis.

On ajoute goutte à goutte à 0°C, 0,3 ml de chlorothioformiate de 0 para tolyle à un mélange de 0,2 g d'alcool-ester obtenu au stade précédent et de 0,16 ml de pyridine dans 6 ml de chlorure de méthylène sec. Après 48 heures, à 0°C le mélange réactionnel est adsorbé sur une quantité minimale de silice (≅ 2 g). On chromatographie sur silice en éluant avec de l'hexane à 2 % d'éther. On obtient ainsi 0,383 g de thiocarbonate (F=92-93°C, après recristallisation dans l'hexane).

Stade E: cis-chrysanthémate de méthyle.

Une solution de 0,693 g de thiocarbonate obtenue au stade précédent dans 10 ml de trichloro-1,2,4-benzène est chauffée lentement jusqu'à 140°C, puis maintenue pendant un quart d'heure à cette température. Après refroidissement, la solution est versée directement sur colonne de silice et éluée avec de l'hexane à 2 % d'éther. On recueille 0,323 g de cis-chrysanthémate de méthyle attendu.

**Revendications**

1) Procédé de préparation d'esters d'alcoyle de l'acide cis chrysanthémique, racémiques ou optiquement actifs, répondant à la formule (I):

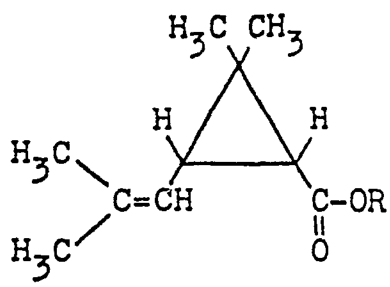

(I)

dans laquelle R représente un radical alcoyle renfermant de 1 à 6 atomes de carbone, caractérisé en ce que l'on traite un composé de formule (II):

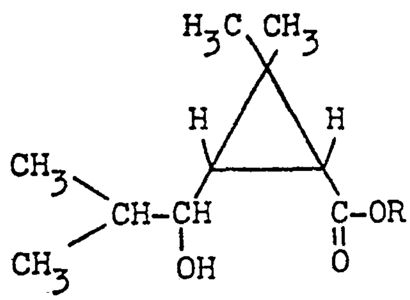

(II)

de configuration cis, racémique ou optiquement actif, dans laquelle R a la signification précitée, par un halogénothioformiate d'aryle de formule:

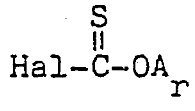

(III)

dans laquelle Hal représente un atome d'halogène et $A_r$ représente un radical aryle renfermant de 6 à 12 atomes de carbone, éventuellement substitué, pour obtenir un composé de formule (IV):

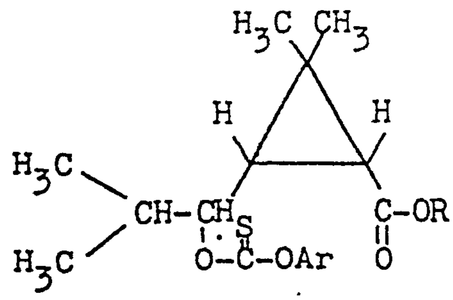

(IV)

de configuration cis, formule (IV) dans laquelle R et $A_r$ ont la signification précitée, que l'on chauffe, le cas échéant au sein d'un solvant organique, pour obtenir un composé de formule (I) attendu.

2) Procédé selon la revendication 1, caractérisé en ce que l'halogénothioformiate d'aryle est le chlorothioformiate de p-tolyle.

3) Procédé selon la revendication 1 ou 2, caractérisé en ce que l'on chauffe le composé de formule (IV) à une température de 100 à 150°C, au sein d'un solvant organique non polaire.

4) Procédé selon la revendication 3, caractérisé en ce que le solvant organique est un dichloro ou un trichlorobenzène.

5) Procédé selon l'une quelconque des revendications 1 à 4, pour la préparation du composé de formule (II), caractérisé en ce que l'on traite le composé de formule (X):

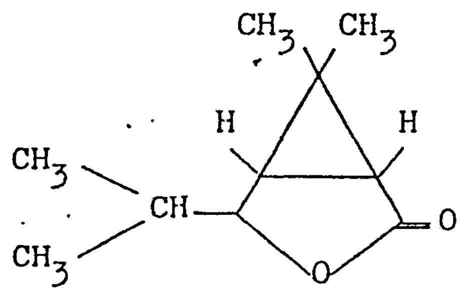

(X)

par un agent basique, puis par un agent d'estérification, obtenir le composé de formule (II).

6) Procédé selon la revendication 5, caractérisé en ce que le traitement par un agent basique du composé de formule (X) est effectué par un hydroxyde alcalin en milieu hydroalcoolique.

7) Procédé selon la revendication 5, caractérisé en ce que l'estérification conduisant aux esters de formule (II) est effectuée in situ, sans isolement de l'acide issu de la saponification du composé de formule (X).

8) A titre de composés industriels nouveaux, les composés de formule (IV), de configuration cis, racémiques ou optiquement actifs, dans laquelle R et $A_r$ sont définis comme à la revendication 1.

## Patentansprüche

1. Verfahren zur Herstellung von racemischen oder optisch aktiven Alkylestern der cis-Chrysanthemumsäure der Formel (I):

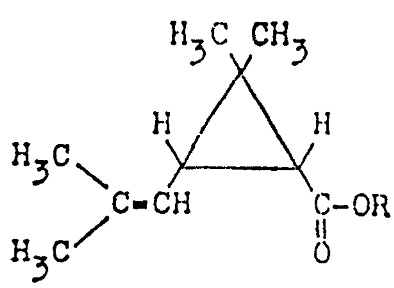

(I)

worin R einen Alkylrest mit 1 bis 6 Kohlenstoffatomen bedeutet, dadurch gekennzeichnet, daß man eine racemische oder optisch aktive Verbindung der Formel (II):

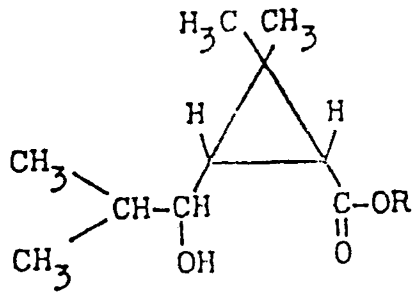

(II)

mit cis-Konfiguration, worin R die angegebene Bedeutung besitzt, mit einem Arylhalogenothioformiat der formel (III):

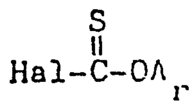

(III)

worin Hal ein Halogenatom bedeutet und $A_r$ einen gegebenenfalls substituierten Arylrest mit 6 bis 12 Kohlenstoffatomen darstellt, behandelt, um eine Verbindung der Formel (IV):

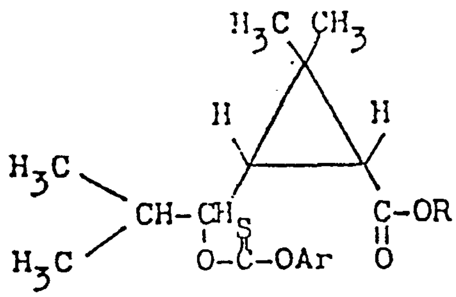

(IV)

mit cis-Konfiguration, worin R und $A_r$ die vorstehende Bedeutung besitzen, zu erhalten, die man gegebenenfalls in dem Medium eines organischen Lösungsmittels erwärmt, um eine erwartete Verbindung der Formel (I) zu erhalten.

2. Verfahren gemäß Anspruch 1, dadurch gekennzeichnet, daß das Arylhalogenothioformiat das p-Tolylchlorothioformiat ist.

3. Verfahren gemäß Anspruch 1 oder 2, dadurch gekennzeichnet, daß man die Verbindung der Formel (IV) auf eine Temperatur von 100 bis 150°C in dem Medium eines nicht-polaren organischen Lösungsmittels erwärmt.

4. Verfahren gemäß Anspruch 3, dadurch gekennzeichnet, daß das organische Lösungsmittel ein Dichlor- oder Trichlorbenzol ist.

5. Verfahren gemäß einem der Ansprüche 1 bis 4 zur Herstellung der Verbindung der Formel (II), dadurch gekennzeichnet, daß man die Verbindung der Formel (X):

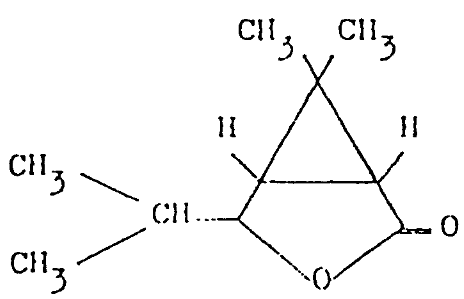

(X)

mit einem basischen Mittel, danach mit einem Veresterungsmittel behandelt um die Verbindung der Formel (II) zu erhalten.

6. Verfahren gemäß Anspruch 5, dadurch gekennzeichnet, daß die Behandlung mit einem basischen Mittel der Verbindung der formel (X) mit einem Alkalihydroxyd in wäßrigalkoholischem Milieu durchgeführt wird.

7. Verfahren gemäß Anspruch 5, dadurch gekennzeichnet, daß die zu den Estern der Formel (II) führende Veresterung in situ ohne Isolierung der aus der Verseifung der Verbindung der formel (X) hervorgegangenen Säure durchgeführt wird.

8. Als neue industrielle Verbindungen, die racemischen oder optisch aktiven Verbindungen der Formel (IV) mit cis-Konfiguration, worin R und $A_r$ wie in Anspruch 1 definiert sind.

**Claims**

1) Prepararation process for racemic or optically active alkyl esters of cis chrysanthemic acid, answering to the formula (I):

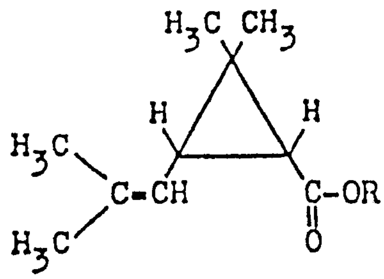

(I)

in which R represents an alkyl radical containing from 1 to 6 carbon atoms, characterized in that a compound with the formula (II):

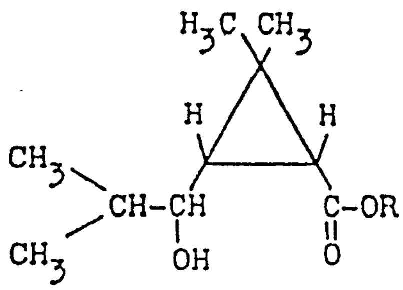

(II)

of cis configuration, racemic or optically active, in which R has the previously given significance, is treated by an aryl halogenothioformate with the formula:

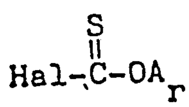

(III)

in which Hal represents a halogen atom and $A_r$ represents an aryl radical containing from 6 to 12 carbon atoms, possibly substituted, so as to obtain a compound with the formula (IV):

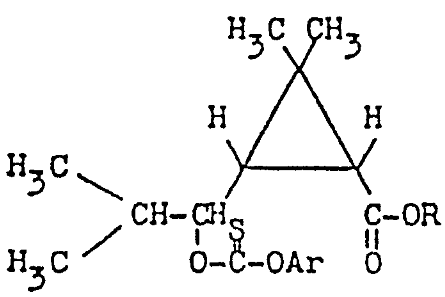

(IV)

of cis configuration, in which formula (IV) R and $A_r$ have the previously given significance, which is heated, if necessary in an organic solvent, so as to obtain an expected compound with the formula (I).

2) Process according to claim 1, characterized in that the aryl halogenothioformate is p-tolylchlorothioformate.

3) Process according to claim 1 or 2, characterized in that the compound with the formula (IV) is heated to a temperature of 100 to 150°C, in a non-polar organic solvent.

4) Process according to claim 3, characterized in that the organic solvent is a dichloro- or trichlorobenzene.

5) Process according to any one of the claims 1 to 4, for the preparation of the compound with the formula

(II), characterized in that the compound with the formula (X):

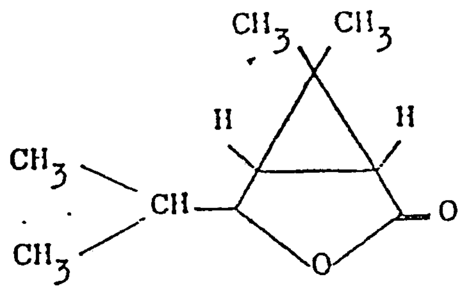

(X)

is treated by a basic agent, when by an esterification agent, so as to obtain the compound with the formula (II).

Process according to claim 5, characterized in that the treatment by a basic agent of the compound with the formula (X) is carried out by alkaline hydroxide in a water-alcohol medium.

7) Process according to claim 5, characterized in that the esterification leading to the esters with the formula (II) is carried out in situ, without isolating the acid resulting from the saponification of the compound with the formula (X).

8) As new industrial compounds, the compounds with the formula (IV), of cis configuration, racemic or optically active, in which R and $A_r$ are defined as in claim 1.